(19) Europäisches Patentamt

European Patent Office

Office européen des brevets

(11) EP 0 948 947 A2

(12) **EUROPEAN PATENT APPLICATION**

(43) Date of publication:
13.10.1999 Bulletin 1999/41

(51) Int. Cl.⁶: **A61F 2/66**, A61F 2/74

(21) Application number: 99302709.3

(22) Date of filing: 07.04.1999

(84) Designated Contracting States:
AT BE CH CY DE DK ES FI FR GB GR IE IT LI LU
MC NL PT SE
Designated Extension States:
AL LT LV MK RO SI

(30) Priority: 07.04.1998 GB 9807386

(71) Applicant: O'Byrne, Michael
Oxford (GB)

(72) Inventor:
Boender, Jacob Quintus Laurens Anthony
Marcham, Oxfordshire OX13 6P2 (GB)

(74) Representative:
Spencer, Michael David, Dr. et al
Bromhead & Co.,
19 Buckingham Street
London WC2N 6EF (GB)

(54) **Hydraulic prosthetic joint**

(57)    A hydraulic prosthetic joint 10 having a silicone-based hydraulic agent with high viscosity and a viscoelastic response so that the joint is self-aligning and stable with satisfactory movement control. The joint 10 may be constructed in pivotal configuration for use as, for example, an ankle joint 10, in which different angles of plantarflexion and dorsiflexion are provided and the hydraulic flow is modified to balance the plantarflexion and the dorsiflexion moments. The joint may also be configured as a rotary joint 41.

FIGURE 5

Printed by Xerox (UK) Business Services
2.16.7/3.6

## Description

The present invention relates to prosthetic joints.

Prosthetic joints are commonly hydraulic, so that the movement of the joint about its axis, or the movement of parts of the joint relative to each other, is controlled by the damping properties of a viscous hydraulic fluid. The restricted viscous displacement of the fluid provides the control, and this may operate in conjunction with elastic elements such as springs or rubber components. Hence viscous and elastic properties can be provided, which is desirable for acceptable simulation of joint function.

The hydraulic fluid is typically a hydrocarbon hydraulic oil, having a low viscosity, typically below 50 centistokes. To achieve sufficient viscous effect from such a substance it is necessary for the joint to be relatively bulky, which is undesirable, for cosmetic reasons, for example. Smaller joint constructions are not powerful enough, and are also subject to overheating of the oil due to movement in a confined space. Furthermore, the oil lacks an elastic response, which makes it suitable for some prosthetic applications, such as artificial knee joints, but less suitable for other types of artificial joint, such as ankles, which ideally are provided with some elastic properties to enable the joint to be self-realigning and hence resume its original shape and position.

Effective control of a prosthetic ankle joint requires the joint to assume a flat foot position under a wide range of circumstances. Previously proposed prosthetic ankle joints have used leaf springs or compliant rubber in attempts to achieve this. However, such compliant elastic components tend to cause a residual bending moment in the ankle joint shank, which leads to increased difficulty in controlling downhill walking. Alternatively, a hydraulic design has been proposed [*PLEASE PROVIDE REFERENCE FOR MAUCH*] in which dorsiflexion of the joint during walking is permitted until the shank reaches a vertical position and causes closure of a hydraulic chamber to prevent further movement. However, difficulties with hydraulic sealing have caused problems with this device.

An aim of the present invention is to address these shortcomings.

Accordingly, the present invention is directed to a hydraulic prosthetic joint in which the hydraulic agent is silicone-based. Silicone can provide a viscoelastic response which is well-suited to give natural control and movement of a prosthetic joint

Advantageously the hydraulic agent is a silicone fluid. High viscosity can be achieved with a silicone fluid, which offers a number of advantages, including the use of more compact joint constructions, and lower machining tolerance requirements in joint manufacture.

Preferably the silicone fluid has a viscosity of at least 10,000 centipoise.

Alternatively, the hydraulic agent is a composite comprising silicone rubber solid and silicone fluid. The silicone rubber solid provides a primarily elastic response and the silicone fluid provides a primarily high viscosity response, so that an overall viscoelastic response can be achieved, and tailored to meet the demands of a specific joint.

Preferably the composite hydraulic agent comprises silicone rubber solid particles suspended in a silicone fluid matrix. Such a construction emphasises the viscosity of the viscoelastic response.

Advantageously the smallest dimension of the particles is not less than 0.3 mm.

Alternatively the composite hydraulic agent comprises porous flexible silicone rubber solid filled with silicone fluid. Such a construction emphasises the elasticity of the viscoelastic response.

In a preferred embodiment the hydraulic agent further comprises magnetic particles. This offers the advantage that the flow of the hydraulic agent can be controlled by the application of a magnetic field to align the magnetic particles, which can increase the dynamic viscosity.

A second aspect of the present invention is directed to a hydraulic prosthetic joint comprising a base section, an upper section pivotally mounted to the base section via pivot means, and a hydraulic chamber defined by the upper section and the base section and filled with a hydraulic agent. This construction is suitable for a variety of joints.

Preferably the prosthetic joint further comprises a body disposed within the hydraulic chamber. This has the effect of partly partitioning the hydraulic chamber so that the hydraulic agent must flow around the body.

Advantageously the body is attached to the base section such that a space is provided between the upper surface of the body and the underside of the upper section. The space acts as a hydraulic passage so that the hydraulic agent can flow between different parts of the hydraulic chamber via the passage.

Preferably the body is an upright member situated substantially centrally in the hydraulic chamber. This provides a broadly symmetric distribution of hydraulic agent within the hydraulic chamber so that movement of the prosthetic joint is well-balanced.

In a preferred embodiment the pivot means is a ball joint. Ball joints provide a simple, low friction, low wear pivot means.

Preferably the ball joint uses ball bearings.

Advantageously the base section is provided with a shoulder on its upper edge to contain the upper section and define its pivotal movement.

Preferably a hydraulic seal is disposed between the upper section and the base section.

Advantageously the upper section is provided with a projecting lip on its lower surface. This increase the area acting on the hydraulic agent and hence increases displacement and compression of the hydraulic agent during pivoting of the ankle joint, thus producing a greater response.

Advantageously the hydraulic prosthetic joint is further provided with an externally adjustable valve to control flow of the hydraulic agent over the top of the body. Thus the flow can be set at a rate most appropriate for a particular user's requirements and characteristics, so that the joint is responsive under a variety of conditions.

Preferably the hydraulic prosthetic joint is further provided with a manual lock to constrain the permitted pivotal movement. This provides a safety feature useful, for example, when the user is driving.

In a preferred embodiment the body has an asymmetric profile in a plane substantially orthogonal to the pivot axis of the upper section. Thus a different resistance to flow of the hydraulic agent occurs for the different directions of movement, so that the joint can be biased to pivot in one direction preferentially.

Advantageously the upper section can pivot through a greater angle in one direction than in the other direction with respect to the position equivalent to the joint being at rest. This feature can simulate the movement of a real joint, in which movement in one direction is greater than in another direction.

In a preferred embodiment the hydraulic prosthetic joint is an ankle joint.

Advantageously the base section of the ankle joint is adapted for connection to a prosthetic foot.

Advantageously the upper section of the ankle joint is adapted for connection to a lower leg.

Preferably the greater pivot angle corresponds to movement of the prosthetic ankle producing plantarflexion of a prosthetic foot. This construction allows simulation of a real ankle joint, in which plantarflexion is greater than dorsiflexion.

Preferably the lower surface of the upper section is a slanted distal plane. This construction provides the desired difference in plantarflexion and dorsiflexion without the need for additional components within the joint.

Advantageously the body has a profile which causes less resistance to flow of the hydraulic agent for rotation of the upper section in the direction of rotation corresponding to plantarflexion of a prosthetic foot. This compensates for a net dorsiflexion moment resulting from the asymmetry of the foot, and hence improves stability.

Preferably the upper section is free to tilt from side to side. This provides the advantage that the user can more easily negotiate cambers and uneven ground.

Advantageously the underside of the upper section is provided with a protrusion and the upper surface of the body is provided with a co-operating recess such that the recess is of sufficient size to permit sideways movement of the protrusion within it. Such a configuration allows the desired tilting movement but constrains it to an appropriate angle by restricting the movement of the upper section in relation to the base member.

Alternatively, the underside of the upper section is provided with a recess and the upper surface of the body is provided with a co-operating protrusion such that the recess is of sufficient size to permit sideways movement of the protrusion within it.

Preferably the co-operating protrusion and recess further permit slight rotation of the upper section with respect to the base section about the vertical axis of the prosthetic ankle joint. This simulates the rotation of a foot about an ankle.

Preferably the hydraulic agent is silicone-based. The viscoelastic response of the hydraulic agent is well-suited to an ankle joint, and provides self-alignment of a prosthetic foot used in conjunction with the ankle joint.

A third aspect of the present invention is directed to a hydraulic prosthetic joint which is a rotary joint comprising a housing defining a hydraulic chamber and a rotor member disposed within the hydraulic chamber and free to rotate therein such that the angle through which the rotor member can rotate is limited. The permitted angle can be selected to approximate natural movement of the particular rotary joint required.

Advantageously the housing is provided with a plurality of protrusions projecting into the hydraulic chamber and the rotor member is provided with a plurality of extensions to cooperate with the protrusions to limit the permitted angle through which the rotor member can rotate.

Alternatively the housing is provided with a plurality of recesses and the rotor member is provided with a plurality of extensions extending into the recesses to cooperate therewith to limit the permitted angle through which the rotor member can rotate.

Advantageously the profile of the protrusions, extensions and/or recesses in the plane orthogonal to the axis of rotation of the rotor member is asymmetric so that the resistance to flow of the hydraulic agent is greater for one direction of rotation than for the other. Thus the joint can rotate more easily in one direction than in the other, so natural movement can be better simulated.

Preferably the hydraulic agent is silicone-based. This gives the advantage that the viscoelastic response of the agent can be exploited to simulate natural movement of the joint, and provide self-alignment.

Examples of prosthetic joints made in accordance with the present invention will now be described with reference to the accompanying drawings, in which:

Figure 1 shows a sectional side view of a prosthetic ankle joint made in accordance with the present invention.

Figure 2 shows a sectional front view of the joint of Figure 1;

Figure 3 shows a sectional side view of the joint of Figure 1 in a pivoted position;
Figure 4 shows schematic sectional views of alternative embodiments of the central portion of the joint of Figure 1;
Figure 5 shows a sectional side view of the joint of Figure 1 in use in conjunction with a prosthetic foot;
Figure 6 shows sectional front views of a further embodiment of the joint of Figure 1;
Figure 7 shows sectional views of embodiments of a rotary joint made in accordance with the present invention; and
Figure 8 shows schematic views of embodiments of a silicone hydraulic agent made in accordance with the present invention.

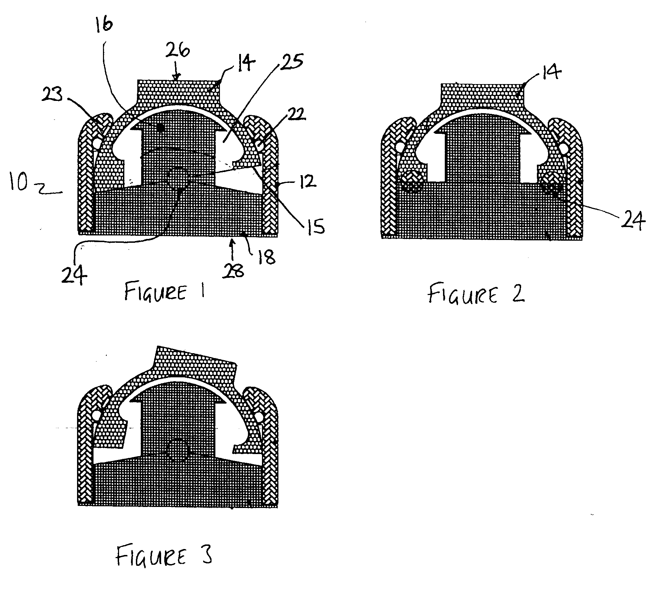

A prosthetic ankle joint 10, as shown in Figure 1, is made in the form of a ball and socket joint and comprises an upper section 14 held in a base section comprising an annular housing 12 and a base 18 such that the upper section 14 can rotate within the housing 12. The upper surface 26 of the upper section 14 is adapted for connection to a lower leg. The lower surface 28 of the base 18 is adapted for connection to a prosthetic foot.
The underside of the upper section 14 is substantially hollow, with the lower surface forming a slanted distal plane. The perimeter of the lower surface is provided with an annular lip 15 projecting inwards. The upper rim of the annular housing 12 is provided with a shoulder 23 to guide and support the upper section 14 during rotation within the annular housing 12.
The hollow underside of the upper section 14, the upper side of the base 18 and the walls of the annular housing 12 define a hydraulic chamber 25. An annular hydraulic seal 22 is disposed between the outer surface of the upper section 14 and the shoulder 23 of the annular housing 12.
An upright body 16 is disposed substantially centrally in the hydraulic chamber 25 and mounted on the base 18. Its upper surface is shaped to permit movement of the upper section 14. A space is provided between the upper surface of the upright body 16 and the lower surface of the upper section 14.
The hydraulic chamber 25 contains a hydraulic fluid. Preferably the hydraulic fluid is a hydraulic agent in the form of a high viscosity viscoelastic silicone fluid or a composite material comprising silicone rubber solid and silicone liquid.

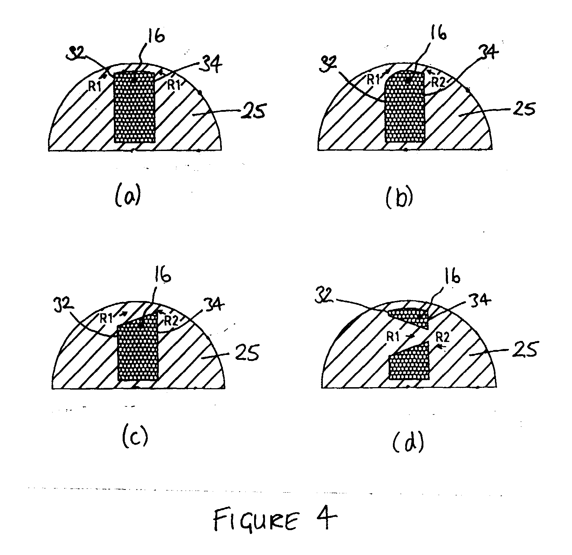

Figure 2 shows a front cross section of the ankle joint 10. Ball bearing joints 24 are positioned at each side of the base 18 to pivotally connect the base member to the upper section 14.
In use, the ankle joint 10 is free to pivot about the ball bearing joints 24. The slanted distal plane of the underside of the upper section 14 permits rotation through a greater angle in one direction than in the other. This feature is used to approximate the natural movement of the foot, in which dorsiflexion, whereby the angle between the foot and the leg is reduced, is more limited than plantarflexion, whereby the angle is increased.
The properties of the silicone hydraulic agent are such that the agent has a relatively high viscosity (typically 10,000 centipoise or more) compared to the low viscosity hydraulic fluids commonly used in hydraulic prostheses, which are typically hydrocarbon oils having viscosities below 50 centistokes. The agent also has elastic properties.
Such a high viscosity viscoelastic agent is ideally suited to use in a prosthetic joint such as an ankle. In use the hydraulic agent causes suitable damping to produce desirable and reliable operation of the joint, and a more realistic motion and control than can be achieved with the low viscosity hydrocarbon oils. Moreover, the ankle joint 10 of Figure 1 is effectively self-aligning because of the elastic properties of the hydraulic agent. Because the agent is viscoelastic, it is less than 100% damping. The elastic properties are such that the agent is able to resume its original position after small amounts of compression and displacement so that the joint can move under the elastic restoring forces to reach a natural position, depending on the position of the user.
The hydraulic agent offers further advantages. For instance, the higher viscosity allows the use of larger hydraulic flow passages within prosthetic joints, which both lowers production costs of such joints due to reduced machining tolerance requirements, and reduces the likelihood of passage blockage. In addition, a smaller volume of the hydraulic agent than of a hydrocarbon oil is required to produce an equivalent effect, so that the joint can be made more compact overall, giving a better cosmetic appearance. Also, the deformation and displacement of smaller volumes of hydraulic agent which occur during movement of the joint are more evenly distributed through the agent as a whole, and hence cause less localised heating and reduced thermal breakdown of the agent as compared to a hydrocarbon oil.

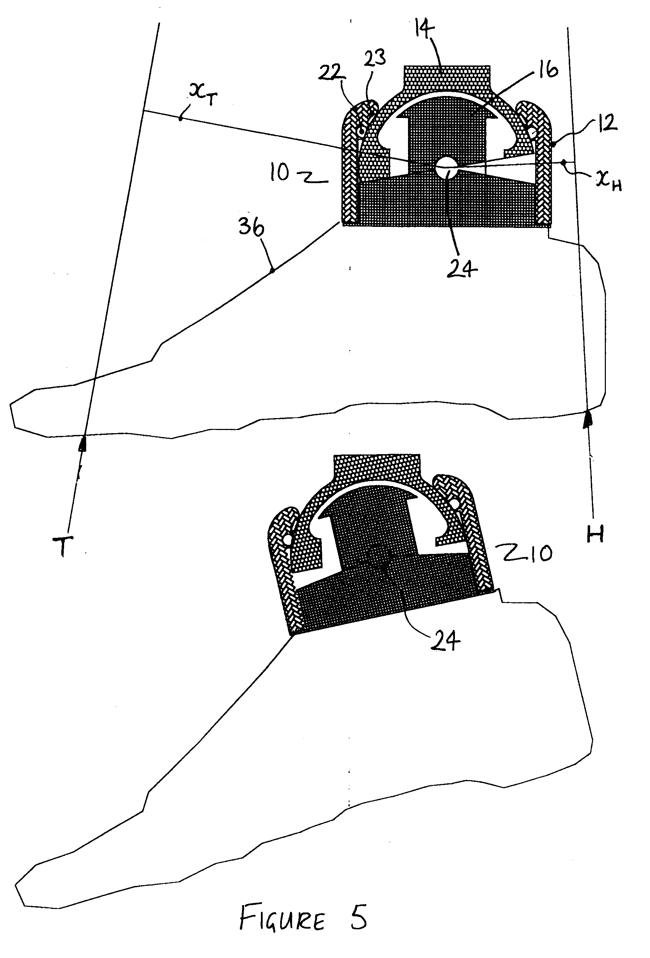

Figure 4 shows a selection of possible configurations of the upright body 16 within the hydraulic chamber 25. When the ankle joint 10 is rotated about the axis of the ball bearing joints 24, the hydraulic agent flows within the chamber, some of it passing over the top of the upright body 16, as indicated by the arrows R1 and R2. Flow of the agent is facilitated by the lip 15 on the upper section 14, which causes increased displacement and distortion of the agent. Figure 4a shows an upright body 16 which is symmetrical with respect to the surfaces 32 and 34, positioned to the back and front of the ankle joint 10. Hence the flow R1 is the same for rotation of the ankle joint 10 in either direction. However, movement of the ankle joint 10 can be made to more naturally approximate a real ankle joint if there is preferential flow in one direction. The reason for this is illustrated in Figure 5, which shows a cross-sectional side view of the ankle joint 10, in use with a prosthetic foot 36.

During walking, at heel strike a force H acting for a time $t_H$ at a distance $x_H$ from the ball bearing joint 24 causes a moment in the plantar flexion direction $M_H = Hx_H$ about the ball bearing joint 24. Later in the user's gait, the toe-off force T acts for a time $t_T$ at a distance $x_T$ from the ball bearing joint 24, causing a moment $M_T = Tx_T$ in the plantar flexion direction. The dimensions of the foot and ankle are such that $x_T > x_H$. However, the forces H and T are typically similar in magnitude and duration, so that during walking a net moment occurs in the dorsiflexion direction. This net moment is generally undesirable in providing stability and natural foot movement, but it can be offset by biasing the flow of the hydraulic agent so that plantarflexion occurs more easily than dorsiflexion. This can be achieved by providing an asymmetric upright body 16, as shown in Figures 4b, c and d. In each of these configurations the surfaces 32 and 34 are shaped so that the hydraulic agent can flow more readily over the surface 32 than the surface 34. Hence the resistance to the flow R1 is less than the resistance to the flow R2.

The volume of the upright body 16 can be adjusted to suit the requirements of the user. For example, a larger upright body 16 reduces the volume of the chamber 25 and hence reduces the flow. This arrangement is more suitable for a heavier user.

Note that in Figure 4 the ankle joint 10 is viewed from the opposite side to that shown in Figure 1 to 3.

Additionally, an external valve (not shown) may be provided to adjust the flow and thus adapt the ankle joint 10 to better suit particular user requirements. Advantageous operation of the ankle joint 10 is given when the flow is balanced to make the ankle joint 10 responsive to uphill walking, downhill walking and level-ground walking.

Furthermore, a manual lock (not shown) can be provided to constrain the motion of the ankle joint 10, which can be beneficial, for example, by making driving safer.

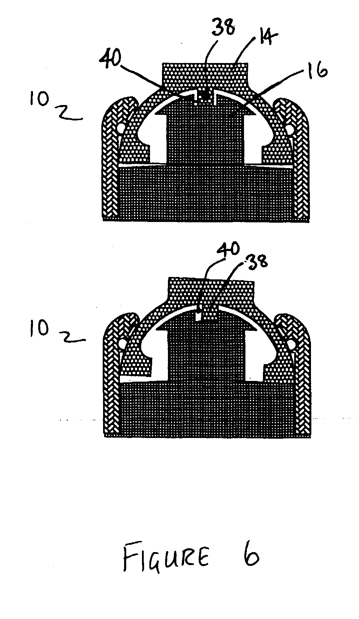

Figure 6 shows an alternative embodiment of the ankle joint 10 of Figure 1. The underside of the upper member 14 is provided with a protrusion 38. The upper surface of the upright body 16 is provided with a recess 40 to co-operate with the protrusion 38 and provide a partial partition between the two sides of the hydraulic chamber. The recess 40 is larger than the protrusion 38 so that the protrusion 38 has restricted movement within the recess 40. This movement allows for a small side-to-side tilt of the ankle joint 10 so that the user can better negotiate cambers and uneven ground. The elastic properties of the hydraulic agent restore the foot to a more horizontal position when it is lifted from the uneven ground. Furthermore, if sufficient tolerance is provided in the cooperation of the protrusion 38 and recess 40, a small amount of rotational movement about the central vertical axis of the ankle joint 10 can be accommodated. Obviously it is possible to reverse the position of the protrusion 38 and recess 40 such that the protrusion 38 is on the upright body 16 and the recess 40 is in the upper member 14.

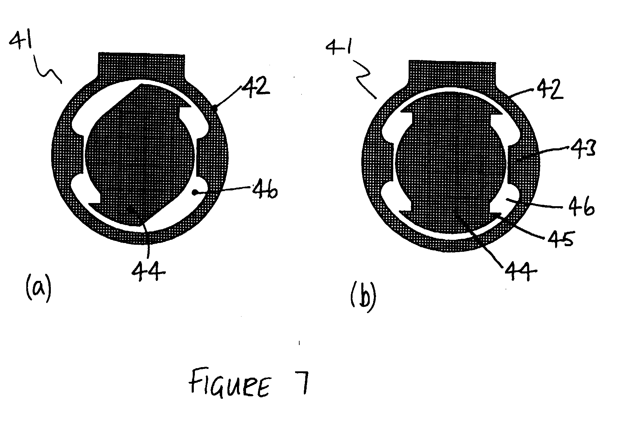

Figure 7 shows two alternative embodiments of a rotary hydraulic joint 41 with the hydraulic agent of the present invention. A housing 42 forms a hydraulic chamber 46. Protrusions 43 extend from the housing 42 into the chamber 46. A rotor member 44 is disposed within the housing 46 and rotate therein. The rotor member 44 is provided with extensions 45 which limit the rotation angle of the joint 41 by abutting the protrusions 43 at the extremes of position of the joint 41. The embodiment of Figure 7b shows a rotor member 44 which is symmetrical with respect to its central rotation axis, so that resistance flow of the hydraulic agent is the same for both directions of rotation. Figure 7a shows an embodiment having an asymmetric rotor member 44, in which flow is preferentially biased for anticlockwise rotation of the rotor member 44. This modification may be appropriate for some rotary joints, so that a more natural movement is simulated.

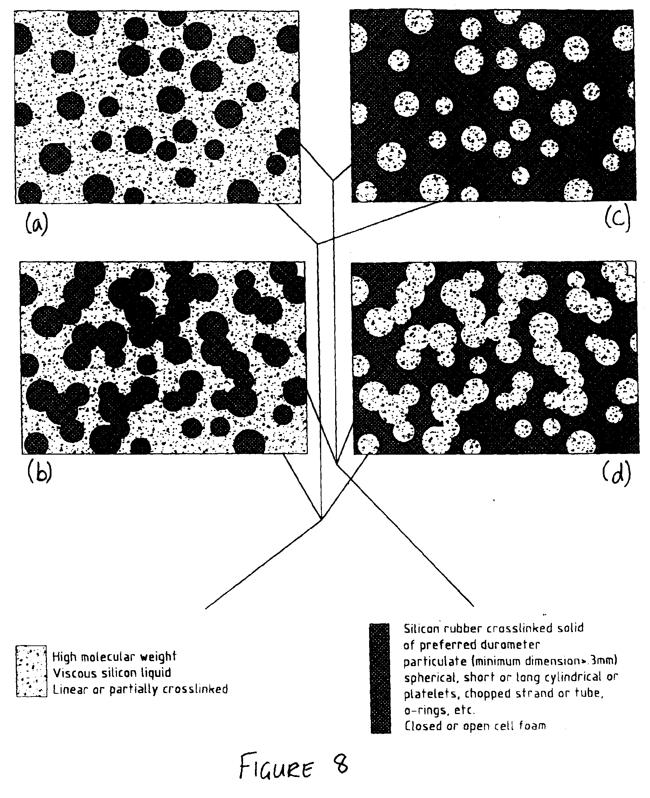

Figure 8 shows alternative embodiments of the hydraulic agent used in the present invention. The hydraulic agent is silicone-based, has a high molecular weight, and a viscosity of at least 10,000 centipoise. A silicone fluid may be used, being linear or partially cross-linked, or alternatively a composite agent of cross linked solid silicone rubber particles and silicone fluid. A composite agent gives an improved viscoelastic response. An agent comprising solid silicone rubber particles suspended in a silicone fluid matrix (Figures 8a and 8b) tends to emphasise the viscosity properties of the agent over the elastic properties. The particles may for example, be spherical, cylindrical, tube-shaped, ring-shaped or platelets. Preferably the smallest dimension of the solid particles is not less than 0.3 mm. Conversely, an agent comprising a porous flexible silicone rubber solid filled with silicone liquid (Figures 8c and 8d) emphasises the elastic properties. The solid particles provide elastic resistance to deformation. Further, the shear forces in the agent increase with particle content, thus increasing the viscous resistance to flow. Hence the composite material can be tailored for specific joint requirements.

## Claims

1. A hydraulic prosthetic joint 10, characterised in that the hydraulic agent is silicone-based.

2. A hydraulic prosthetic joint 10 characterised in that it comprises a base section 12,18, an upper section 14 pivotally mounted to the base section 12,18 via pivot means 24, and a hydraulic chamber 25 defined by the upper section 14 and the base section 12,18 and filled with a hydraulic agent.

3. A hydraulic prosthetic joint 10 according to claim 2, characterised in that it further comprises a body 16 disposed within the hydraulic chamber 25.

4. A hydraulic prosthetic joint 10 according to claim 3, characterised in that the body 16 has an asymmetric profile in a plane substantially orthogonal to the pivot axis of the upper section 14.

5. A hydraulic prosthetic joint 10 according to any of claims 2 to 4, characterised in that the upper section 14 can pivot through a greater angle in one direction than in the other direction with respect to the position equivalent to the joint being at rest.

6. A hydraulic prosthetic joint 10 according to any of claims 2 to 5, in which the upper section 14 is free to tilt from side to side.

7. A hydraulic prosthetic joint 10 according to any of claims 2 to 6, characterised in that it is for use as an ankle joint.

8. A hydraulic prosthetic joint 10 according to any of claims 2 to 7, characterised in that the hydraulic agent is silicone-based.

9. A hydraulic prosthetic joint for use as a rotary joint 41 characterised in that it comprises a housing 42 defining a hydraulic chamber 46 and a rotor member 44 disposed within the hydraulic chamber 46 and free to rotate therein such that the angle through which the rotor member 44 can rotate is limited.

10. A hydraulic prosthetic joint 41 according to claim 9, characterised in that the hydraulic agent is silicone-based.

FIGURE 1

FIGURE 2

FIGURE 3

FIGURE 4

FIGURE 5

FIGURE 6

FIGURE 7

(a)

(c)

(b)

(d)

High molecular weight
Viscous silicon liquid
Linear or partially crosslinked

Silicon rubber crosslinked solid
of preferred durometer
particulate (minimum dimension>.3mm)
spherical, short or long cylindrical or
platelets, chopped strand or tube,
o-rings, etc.
Closed or open cell foam

FIGURE 8